# EUROPÄISCHE PATENTSCHRIFT

(11) **EP 3 402 546 B1**
(45) Veröffentlichungstag und Bekanntmachung des Hinweises auf die Patenterteilung: **03.03.2021**
(21) Anmeldenummer: 17700312.6
(22) Anmeldetag: 05.01.2017
(51) Int. Cl.: A61M 1/16, A61M 1/36, B01L 3/02, A61M 5/31, A61M 5/32

(54) **VORRICHTUNG UND VERFAHREN ZUR PROBENNAHME AUS EINEM FLUIDFÜHRENDEN SYSTEM**
DEVICE AND METHOD FOR DRAWING A SAMPLE FROM A FLUID-CONDUCTING SYSTEM
DISPOSITIF ET PROCÉDÉ DE PRÉLEVEMENT D'ÉCHANTILLONS SUR UN SYSTÈME CONDUCTEUR DE FLUIDE

(30) Priorität: 15.01.2016 DE 102016000370
(43) Veröffentlichungstag der Anmeldung: 21.11.2018
(73) Patentinhaber: Fresenius Medical Care Deutschland GmbH, 61352 Bad Homburg (DE)
(72) Erfinder: HAECKER, Juergen, 61267 Neu-Anspach (DE)
(74) Vertreter: Herrmann, Uwe
(86) Internationale Anmeldenummer: PCT/EP2017/000007
(87) Internationale Veröffentlichungsnummer: WO 2017/121631

(56) Entgegenhaltungen:
- WO-A1-96/21393
- WO-A1-97/45714
- WO-A2-2006/039310
- DE-B3-102006 062 982
- US-A1- 2009 216 174
- US-B1- 6 537 257

## Beschreibung

Die vorliegende Erfindung betrifft eine Vorrichtung sowie ein Verfahren zur Probennahme aus einem fluidführenden System. Die vorliegende Erfindung betrifft des Weiteren ein Blutbehandlungsgerät.

Aus dem Stand der Technik ist es bekannt, aus fluidführenden Systemen, wie beispielsweise aus extrakorporalen Kreisläufen von Blutbehandlungsgeräten Proben z.B. manuell mittels einer Spritze zu nehmen. Dazu ist an dem Schlauchsystem eine Probennahmestelle vorgesehen, die beispielsweise als Septum, Luer-Anschluss, T-Stück oder 3-Wege-Hahn ausgeführt ist.

Ein Vorteil eines Septums gegenüber den anderen genannten Probennahmevorrichtungen besteht darin, dass dieses strömungsgünstig im fluidführenden System integriert werden kann und auch bei der Probennahme selbst Vorteile gegenüber den anderen genannten Vorrichtungen aufweist. So kann mittels einer Kanüle leicht ein eigensicher abgedichteter Zugang zum Fluid bzw. zum Inneren des fluidführenden Systems hergestellt werden. Auch nach dem Herausziehen der Kanüle tritt keine Flüssigkeit aus, da sich das Septum selbsttätig verschließt. Das Septum besitzt somit eine Konnektions- und auch eine Ventilfunktion.

In der klinischen Praxis erfolgt die Probennahme von Hand mittels einer Spritze durch das Septum. Dies bringt den Nachteil mit sich, dass die behandelnde Person einem gewissen Verletzungsrisiko verbunden mit einer Infektionsgefahr ausgesetzt ist.

Aus der US 6,537,257 B1 ist eine Spritze bekannt, deren Spitze bei Nichtbenutzung von einer Hülse umgeben ist, die ein Septum aufweist, das bei der Nutzung der Spritze durchstochen wird. Die US 2009/0216174 A1 offenbart einen mehrlumigen Katheter, wobei ein Lumen des Katheters zum Zwecke der Spülung durch eine Abdeckkappe verschließbar ist.

Die US 2013/0211330 A1 offenbart eine Vorrichtung zur Injektion mit einer in einem Gehäuse bewegbar aufgenommen Kartusche und mit einer im unbenutzten Zustand durch ein Septum verdeckten Nadel. Zur Injektion wird die Kartusche auf die Nadel zubewegt, wobei die Nadel das Septum durchdringt.

Die US 7,594,895 B2 offenbart eine mehrlumige Perfusionsvorrichtung. Aus der US 7,608,042 B2 ist eine Vorrichtung zur automatisierten Blutprobennahme bekannt, die zur Überwachung der Blutzusammensetzung eines Patienten dient. Das Blut wird abgenommen und analysiert und der gemessene Parameterwert wird angezeigt.

Die WO 96/21393 A1 offenbart eine Vorrichtung zur Entnahme von Blutproben. Zudem ist aus der WO 97/ 45714 A1 eine an einer Schlauchleitung angebrachte Vorrichtung zur Entnahme von Blutproben aus der Schlauchleitung bekannt. Weiterhin ist aus der DE 10 2006 062982 B3 eine Vorrichtung zum Nehmen von Proben aus

Systemen mit flexiblen Wandungen und zum Eintragen von Fluiden in solche und aus Probennehmern/Fluidgebern bekannt.

Der vorliegenden Erfindung liegt die Aufgabe zugrunde, eine Vorrichtung der eingangs genannten Art dahingehend weiterzubilden, dass das Verletzungsrisiko bei der Probennahme gegenüber der bekannten Vorgehensweise verringert ist.

Diese Aufgabe wird durch eine Vorrichtung mit den Merkmalen des Anspruchs 1 gelöst.

Danach ist vorgesehen, dass die Vorrichtung wenigstens einen Adapter aufweist, der wenigstens ein Septum und wenigstens eine relativ zu dem Septum verschiebbare Kanüle aufweist, wobei die Kanüle derart verschiebbar ist, dass diese in einer Probennahmeposition das Septum durchdringt und in einer Spülstellung das Septum nicht durchdringt und wobei der Adapter wenigstens einen Spülbereich für ein Spülmedium aufweist, der so angeordnet ist, dass dieser in der Spülstellung der Kanüle mit dem Innenraum der Kanüle in Fluidverbindung steht. Des Weiteren weist die Vorrichtung wenigstens einen Aufnahmebereich mit Arretierungsmitteln für das fluidführende System auf, durch die das fluidführende System relativ zu dem Adapter in einer definierten Position fixiert werden kann.

Durch die vorliegende Erfindung ist es möglich, aus dem fluidführenden System, das sich in dem genannten Aufnahmebereich der Vorrichtung befindet, eine Probe zu entnehmen, ohne dass ein Verletzungsrisiko besteht. Nach dem Einsetzen des fluidführenden Systems in den Aufnahmebereich wird die Kanüle manuell oder mittels eines angetriebenen Verschiebemechanismus so verschoben, dass sie in die Probennahmeposition gelangt, das heißt in die Position, in der sie mit ihrem Einlass mit dem Inneren des fluidführenden Systems in Fluidverbindung steht.

In einer bevorzugten Ausgestaltung der Erfindung weist das fluidführende System seinerseits ein Septum auf, sodass insgesamt in diesem Fall zwei Septen vorgesehen sind, von denen eines Bestandteil des fluidführenden Systems ist und das andere Bestandteil des genannten Adapters der Vorrichtung.

Durch die Arretierungsmittel weist das fluidführende System und vorzugsweise dessen Septum eine feste definierte Position relativ zum Adapter und damit auch relativ zu der Kanüle auf.

Der Begriff "Adapter" ist allgemein zu verstehen und umfasst jede beliebige Einheit, in der das Septum und die Kanüle sowie der Spülbereich angeordnet sind. Eine besondere Anpassung an andere Bestandteile der Vorrichtung kann vorgesehen sein, zwingend erforderlich ist dies jedoch nicht. Vorzugsweise weist der Adapter wenigstens ein Gehäuse auf, in der die Kanüle hin- und her verschiebbar angeordnet ist.

Vorzugsweise ist vorgesehen, dass das fluidführende System relativ zu dem Adapter durch Formschluss fixierbar bzw. fixiert ist. So kann beispielsweise eine mechanisch arretierbare Ankopplung direkt am Septum der Vorrichtung oder auch indirekt über eine Konsole durchgeführt werden. Die Konsole kann beispielsweise als Element eines Blutbehandlungsgerätes ausgeführt sein und den Adapter halten.

Der Adapter selbst verfügt über eine relativ zu seinem Gehäuse und somit auch zu seinem Septum verschiebbare Kanüle. Die Verschiebung der Kanüle erlaubt eine Punktion in der Probennahmeposition und damit eine Fluidverbindung zum fluidführenden System.

In der Spülstellung ist die Kanüle so angeordnet, dass deren Inneres durch ein Spülmedium (z.B. Wasser, Luft oder eine Kalibrierlösung) bzw. durch ein Desinfektionsmedium durchspült bzw. kontaktiert werden kann.

Somit ist eine weitere, separate Fluidverbindung zu mindestens einem weiteren Medium, nämlich zu einem Spülmedium, Desinfektionsmedium etc. möglich.

Die Herstellung dieser Verbindung erfolgt durch das Ein- bzw. Durchtauchen der Kanülenspitze durch das Septum des Adapters in den Spülbereich und damit in einen gegenüber der Umgebung und gegenüber dem fluidführenden System abgedichteten Bereich des Adapters, der vorzugsweise über einen eigenen Fluidanschluss für das Spül- bzw. Desinfektionsmedium verfügt.

Der Aufnahmebereich kann an oder in dem Adapter selbst und/oder an oder in einer Konsole angeordnet sein, auf der der Adapter angeordnet ist. Die Konsole kann Bestandteil eines Blutbehandlungsgerätes, vorzugsweise eines Dialysegerätes sein. Denkbar ist es auch, dass der Aufnahmebereich zwischen dem Adapter und der Konsole angeordnet ist.

Der Verschiebemechanismus zur Verschiebung der Kanüle kann manuell betätigbar sein oder auch so mit einem Antriebselement in Verbindung stehen, dass das Antriebselement den Verschiebemechanismus betätigt. Auf diese Weise ist eine automatisierte Probennahme möglich.

In einer weiteren bevorzugten Ausgestaltung der Erfindung ist vorgesehen, dass das Septum des Adapters das innere Lumen der Kanüle, durch die Probe hindurchströmt, in der Probennahmeposition gegenüber dem Spülbereichfluid dicht abschließt.

Durch die Arretierungsmittel wird vorzugsweise erreicht, dass das Septum des fluidführenden Systems hinter dem Septum des Adapters angeordnet ist, sodass die Kanüle bei dem Verfahren aus ihrer Spülstellung in die Probennahmeposition erst das Septum des Adapters und dann das Septum des fluidführenden Systems durchdringt.

Somit kann durch das Verschieben der Kanüle in deren axialer Richtung das Kanülenende, das heißt der Probenkanaleingang in unterschiedliche, jeweils für sich abgedichtete Kanalbereiche münden. Diese Abdichtung erfolgt vorzugsweise durch hintereinander angeordnete Septen.

Die Vorrichtung kann des Weiteren mit einem Probenaufnahmegefäß und/oder einer Fördereinrichtung und/oder einer Messeinrichtung ausgerüstet sein, wobei die Fördereinrichtung, z.B. im Form einer Vakuumpumpe, zum Entnehmen der Probe durch die Kanüle in der Probennahmeposition dient und die Messeinrichtung zur Messung wenigstens eines Parameters der gezogenen Probe.

Vorzugsweise handelt es sich bei dem fluidführenden System um ein Schlauchsystem bzw. einen Abschnitt eines Schlauchsystems eines Blutbehandlungsgerätes, wie beispielsweise eines Dialysegerätes. In einer bevorzugten Ausgestaltung handelt es sich um einen extrakorporalen Kreislauf oder auch um einen Dialysatkreislauf (bzw. um einen Abschnitt von diesen) eines Blutbehandlungsgerätes und insbesondere eines Dialysegerätes.

In der vordersten Stellung, das heißt in der Probennahmeposition nach dem Durchstechen des Septums des Blutschlauchs bzw. des Dialysatschlauchs kann eine Probe, das heißt z.B. Blut oder Dialysat genommen werden. Nach dem Ansaugen der Blutprobe oder Dialysatprobe wird der Probenkanal gespült.

Hierzu wird die Kanüle in die vom Blutschlauch bzw. Dialysatschlauch oder sonstigen fluidführenden System komplett abgekoppelte Spülposition verfahren. In dieser zweiten Stellung befindet sich die Kanülenspitze in dem Spülbereich, das heißt diesseits der beiden Septen.

Die Spülung der Kanülenaußen- und/oder Innenseite kann beispielsweise mittels gefilterter Sterilluft oder Spül- Kalibrier- oder Desinfektionslösung erfolgen. Dieses kann aus dem Spülbereich in den Probenkanal, das heißt in das innere Lumen der Kanüle eingeleitet werden.

Eine weitere, nicht beanspruchte Vorrichtung zur Probennahme aus einem fluidführenden System weist wenigstens einen Adapter auf und wenigstens einen Aufnahmebereich, in dem sich das wenigstens eine fluidführende System befindet, wobei der Aufnahmebereich mit Arretierungsmitteln für das fluidführende System ausgebildet ist, durch die das fluidführende System relativ zu dem Adapter in einer definierten Position fixiert ist, wobei der Adapter wenigstens eine relativ zu dem fluidführenden System verschiebbar angeordnete Kanüle umfasst, wobei das fluidführende System wenigstens ein Septum aufweist und wobei die Kanüle derart verschiebbar ist, dass diese in einer Probennahmeposition das Septum des fluidführenden Systems durchdringt und in einer Spülstellung diese Septum nicht durchdringt.

In diesem Fall ist somit nur ein einziges Septum vorgesehen, nämlich das des fluidführenden Systems. Der Adapter ist ohne Septum ausgeführt.

Der Adapter kann einen Spülbereich zum Spülen der Kanüle aufweisen jedoch auch ohne einen Spülbereich ausgeführt sein. In letzteren Falle erfolgt die Spülung der Kanüle mittels Luft. Die Kanülenspitze befindet sich in der Spülstellung nicht in einem Spülbereich, sondern ist freiliegend.

Bei dieser Vorrichtung wird das mit wenigstens einem Septum versehene fluidführende System in dem Aufnahmebereich eingebracht und anschließend wird die Kanüle so verfahren, dass diese das Septum durchdringt, so dass die Probe genommen werden kann. Die Kanüle wird anschließend in die Spülstellung zurückverfahren, in der sich die Kanülenspitze nicht mehr innerhalb des fluidführenden Systems befindet, sondern freiliegend oder auch in einem Spülbereich angeordnet ist.

Die vorliegende Erfindung betrifft des Weiteren ein Blutbehandlungsgerät, insbesondere ein Dialysegerät mit wenigstens einer Vorrichtung gemäß einem der Ansprüche 1 bis 9.

Das Blutbehandlungsgerät kann beispielsweise wenigstens eine Steuereinheit aufweisen, die die Vorrichtung so ansteuert, dass die Kanüle zum Zwecke der Probennahme in das in den Aufnahmebereich eingelegte fluidführende System eingeführt wird.

Dies kann vorzugsweise automatisiert erfolgen.

Denkbar ist es, dass die Probennahme und/oder die Spülvorgänge automatisch ein- oder mehrmals zu bestimmten Zeitpunkten oder zu bestimmten Ereignissen, beispielsweise vor und/oder nach einer Blutbehandlung durch das Blutbehandlungsgerät durchgeführt werden.

Wird regelmäßig ein Spülvorgang durchgeführt, besteht keine Gefahr, dass sich der Probenkanal, das heißt das Lumen der Kanüle durch geronnenes Blut zusetzt. Durch das Freispülen der Probennahmeleitung erfolgt auch keine Durchmischung der einzelnen Proben.

Ein wesentlicher Vorteil ist es, dass für den Nutzer keine Verletzungsgefahr an der spitzen Kanüle besteht.

Vorzugsweise sind Mittel vorhanden, die eine Punktierung, das heißt das Verfahren der Kanüle in die Probennahmeposition nur ermöglichen, wenn das fluidführende System in dem Aufnahmebereich angeordnet ist. Weist dieses ein Septum auf, kann vorgesehen sein, dass nur dann eine Probennahme freigegeben wird, wenn ein Septum am Probenadapter angekoppelt ist. Dies kann sowohl durch eine mechanische Nocke bzw. ein mechanisches Element als auch durch einen Sensor bzw. eine Steuereinheit sichergestellt werden.

Wird durch diese Mittel festgestellt, dass sich in dem Aufnahmebereich kein fluidführendes System befindet, ist es denkbar, dass ein Verfahren der Kanüle in die Probennahmeposition durch Sperrmittel verhindert wird.

Die vorliegende Erfindung betrifft des Weiteren ein Verfahren zur Probennahme aus einem fluidführenden Schlauchsystem bzw. einen Abschnitt eines Schlauchsystems eines Blutbehandlungsgerätes mittels einer Vorrichtung mit den Merkmalen nach einem der Ansprüche 1 bis 9 oder mittels einer Blutbehandlungsvorrichtung mit den Merkmalen der Ansprüche 10 oder 11, wobei nach dem Einlegen eines fluidführenden Systems in den Aufnahmebereich die Kanüle manuell oder mittels eines Antriebselementes in das fluidführende System verfahren wird und im Anschluss daran eine Probe aus dem fluidführenden System genommen wird.

Dabei kann das fluidführende System in dem Aufnahmebereich durch eine formschlüssige Verbindung, wie beispielsweise durch eine Verrastung, oder durch eine Klemmverbindung fixiert werden.

Denkbar ist es weiter, dass die Probennahme zeit- oder ereignisgesteuert, jedenfalls bevorzugt automatisiert erfolgt.

In einer weiteren bevorzugten Ausgestaltung der Erfindung ist vorgesehen, dass nach der Probennahme eine Spülung der Kanüle durchgeführt wird. Vorzugsweise wird der Spülvorgang automatisch eingeleitet.

In einer denkbaren Ausführungsform ist vorgesehen, dass das Blutbehandlungsgerät zur Steuerung der Probennahme ein Signal an eine Probennahmesteuerungseinheit sendet. Diese fragt die angeschlossene Messeinrichtung ab, ob diese bereit ist, eine Probe entgegenzunehmen. Ist dies der Fall, wird eine Fluidverbindung wie oben näher beschrieben hergestellt. Diese Verbindung des fluidführenden Systems mit der Vorrichtung ist arretiert. Dies bedeutet, dass das Septum des fluidführenden Systems sowie auch der Adapter sowohl gegeneinander als auch in einer Konsole (sofern vorhanden) formschlüssig fixiert sind. Diese formschlüssige Fixierung kann beispielsweise durch einen oder mehrere Stifte erreicht werden, die in ein oder mehrere Nuten eingreifen.

Ist die Probennahme abgeschlossen, wird die Kanüle von dem fluidführenden System abgekoppelt und wieder in die Spülstellung verfahren.

Als weitere Ausführungsform kann die Kanüle auch hermetisch mittels eines Faltenbalgs in der Spülstellung gegenüber dem Spülraum abgedichtet werden.

Dies hat den zusätzlichen Vorteil, dass keine mit dem Blut in Kontakt kommenden Flächen mit der Atmosphäre in Kontakt kommen können.

Weitere Einzelheiten und Vorteile der Erfindung werden anhand eines in der Zeichnung dargestellten Ausführungsbeispiels näher erläutert.

Es zeigen:
- Figur 1:: eine schematische Schnittansicht durch den Adapter in der Spülstellung und in der Probennahmestellung,
- Figur 2:: unterschiedliche perspektivische Ansichten des erfindungsgemäßen Adapters,
- Figur 3:: eine Längsschnittansicht durch den Adapter gemäß Figur 2 mit eingelegtem fluidführendem System,
- Figur 4:: eine perspektivische Ansicht des Adapters mit Konsole und
- Figur 5:: Schnittansichten durch die Anordnung gemäß Figur 4 mit unterschiedlichen Positionen der Kanüle.

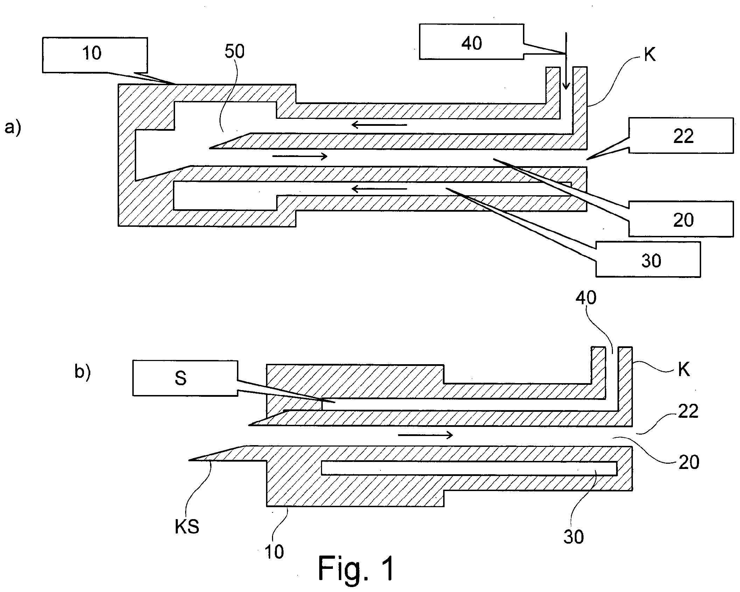

Figur 1a) zeigt mit dem Bezugszeichen 10 das Septum der erfindungsgemäßen Vorrichtung.

In der in Figur 1a) dargestellten Position befindet sich die Kanüle K in der Spülstellung, das heißt die Kanülenspitze KS durchdringt nicht das Septum.

Die Kanüle weist ein inneres Lumen 20 und ein konzentrisch um dieses herum angeordnete äußere Lumen 30 auf.

Das Bezugszeichen 40 kennzeichnet einen Anschluss für ein Spül- oder Desinfektionsmittel, das in Pfeilrichtung in den Spülbereich 50 eintritt. Wie dies aus Figur 1a) hervorgeht, durchströmt das Spülmittel oder Desinfektionsmittel zunächst das äußere Lumen und tritt dann durch die Kanülenspitze KS in das innere Lumen 20 ein. Nach dem Austreten aus dem inneren Lumen 20 an der Stelle 22 wird das Spül- bzw. Desinfektionsmittel verworfen.

Alternativ ist es auch möglich, dass das Bezugszeichen 40 nicht nur den Einlass für das Spül- bzw. Desinfektionsmittel, sondern auch den Auslass bildet.

Soll eine Probe gezogen werden, wird die Kanüle K gemäß Figur 1 nach links verschoben, sodass die Kanüle K mit ihrer Spitze KS das Septum 10 durchstößt, wie dies in Figur 1b) dargestellt ist. In dieser Position wird die Probe gemäß Figur 1b) in Pfeilrichtung durch das innere Lumen 20 z.B. zu einem Probennahmegefäß, zu einer Messeinrichtung etc. geführt. Die Probennahme kann beispielsweise dadurch erfolgen, dass in der Kanüle 20 bzw. in dem inneren Lumen 20 ein Unterdruck angelegt wird.

Das Bezugszeichen S kennzeichnet eine Position, an der die Kanüle K derart mit dem Inneren des Septums 10 in Verbindung steht, dass sich eine fluiddichte Verbindung ergibt. Dies bedeutet, dass keine Fluidverbindung zwischen dem äußeren Lumen 30 und dem inneren Lumen 20 besteht. Dies bringt den Vorteil mit sich, dass mit Sicherheit ausgeschlossen wird, dass ein Spülmedium aus dem äußeren Lumen 30 bei der Probennahme in das innere Lumen 20 und damit in das fluidführende System gelangt.

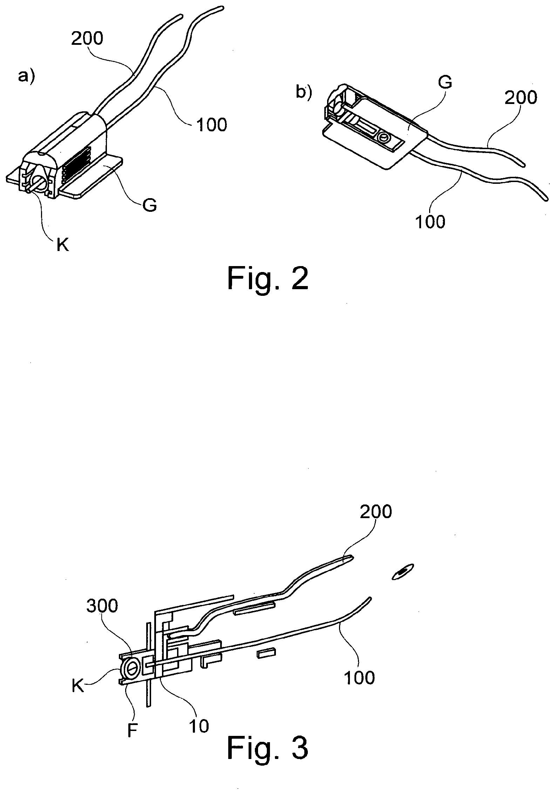

Figur 2a) zeigt in einer perspektivischen Ansicht von schräg oben den Adapter mit Gehäuse G und herausgefahrener Kanüle K. Das Bezugszeichen 100 kennzeichnet einen Schlauch, der mit dem inneren Lumen 20 der Kanüle in Fluidverbindung steht und durch den die genommene Probe z.B. zu einer Analyseneinheit geleitet wird. Das Bezugszeichen 200 kennzeichnet einen Schlauch, mittels dessen Spül- oder Desinfektionsmittel etc. in den Spülbereich 50 eingeleitet werden kann.

Figur 2b) zeigt die Anordnung gemäß Figur 2a) in einer Ansicht von schräg unten mit zurückgezogener Kanüle.

Figur 3 zeigt in einer Längsschnittansicht den Adapter gemäß der Erfindung mit daran dem angekoppelten fluidführenden System F. Das fluidführende System F ist mechanisch in einer bestimmten Position relativ zu dem Adapter fixiert und weist seinerseits ein Septum 300 auf. Beim Bewegen der Kanüle von der Spülstellung in die in Figur 3 dargestellte Probennahmeposition durchdringt die Kanülenspitze zunächst das Septum 10 des Adapters und sodann das Septum 300 des fluidführenden Systems F.

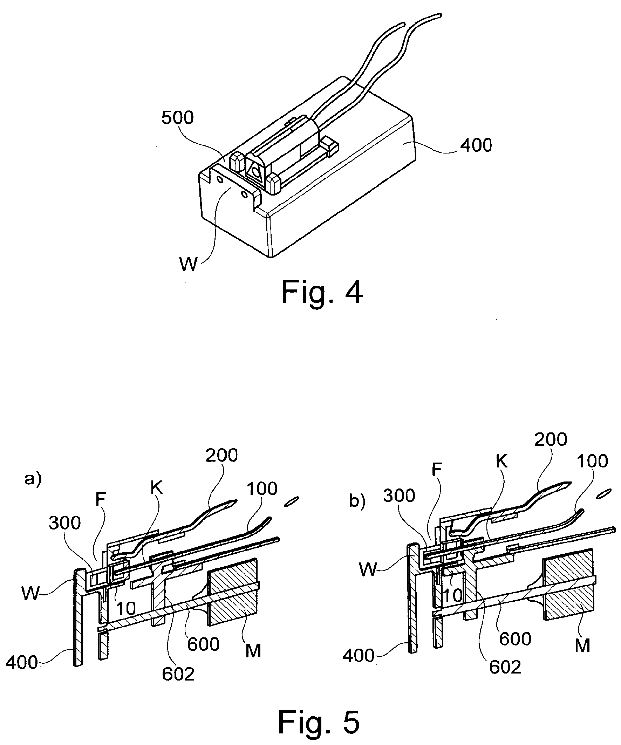

Figur 4 zeigt die Anordnung des Adapters gemäß Figur 2 oder gemäß Figur 3 auf einer Konsole 400. Diese Konsole kann beispielsweise Bestandteil eines Blutbehandlungsgerätes sein. Wie dies aus Figur 4 hervorgeht, befindet sich zwischen der Stirnseite des Adapters und einer Wandung W der Konsole 400 der längliche Aufnahmebereich 500, der zum Einlegen eines Schlauches, z.B. von einem extrakorporalen Blutkreislauf geeignet und bestimmt ist.

Figur 5 zeigt eine Schnittdarstellung durch die Anordnung gemäß Figur 4 mit in dem Aufnahmebereich 500 eingelegtem fluidführendem System F.

Figur 5a) zeigt die Anordnung des fluidführenden Systems zwischen der Wandung W der Konsole 400 und der Stirnseite des Adapters. In dieser Position ist die Kanüle K zurückgezogen und befindet sich in ihrer Spülstellung, in der sie weder das Septum 10 noch das Septum 300 durchdringt. Mit dem Bezugszeichen M ist ein Elektromotor gekennzeichnet, der mit einer Spindel 600 in Verbindung steht, die in Betrieb des Motors die Spindel 600 in eine Drehbewegung versetzt ist. Auf dieser Spindel ist hin- und herverfahrbar der Reiter 602 angeordnet, der seinerseits mit der Kanüle K in Verbindung steht. Durch das Betätigen des Motors kann somit die Kanüle K gemäß Figur 5 nach links oder rechts, das heißt in die Probennahmeposition oder in die Spülstellung verfahren werden.

Figur 5a) zeigt die Kanüle K in der Spülposition und Figur 5b) in der Probennahmeposition.

Durch die vorliegende Erfindung ist es möglich, z.B. aus dem extrakorporalen Blutkreislauf eines Blutbehandlungsgerätes, wie beispielsweise eines Blutbehandlungsgerätes eine Probe zu nehmen, ohne dass für einen Nutzer ein Verletzungsrisiko durch die Spitze der Kanüle besteht.

Des Weiteren kann eine automatisierte Probennahme erfolgen, z.B. getriggert durch eine nicht dargestellte Steuer- oder Regelungseinheit, die in bestimmten Zeitabständen die Kanüle K in die Probennahmeposition verfährt.

Mit dem Schlauch 100 kann ein Probennahmegefäß, eine Messeinrichtung etc. in Verbindung stehen und die genommene Probe analysieren.

Die Probennahme kann zu bestimmten Zeitpunkten erfolgen oder auch bei Eintreten bestimmter Ereignisse, wie z.B. vor und nach einer Blutbehandlung.

## Patentansprüche

1. Vorrichtung zur Probennahme aus einem fluidführenden System, **dadurch gekennzeichnet, dass** die Vorrichtung wenigstens einen Adapter aufweist, der wenigstens ein Septum (10) und wenigstens eine relativ zu dem Septum (10) verschiebbare Kanüle (K) umfasst, wobei die Kanüle (K) derart verschiebbar ist, dass diese in einer Probennahmeposition das Septum (10) durchdringt und in einer Spülstellung das Septum (10) nicht durchdringt und wobei der Adapter wenigstens einen Spülbereich (50) für ein Spülmedium aufweist, der so angeordnet ist, dass dieser in der Spülstellung der Kanüle (K) mit dem Innenraum der Kanüle (K) in Fluidverbindung steht, und wobei die Vorrichtung wenigstens einen Aufnahmebereich (500) mit Arretierungsmitteln für das wenigstens eine fluidführende System (F) aufweist, durch die das fluidführende System (F) relativ zu dem Adapter in einer definierten Position fixierbar ist.

2. Vorrichtung nach Anspruch 1, **dadurch gekennzeichnet, dass** die Arretierungsmittel das fluidführende System (F) relativ zu dem Adapter durch Formschluss fixieren.

3. Vorrichtung nach Anspruch 1 oder 2, **dadurch gekennzeichnet, dass** sich der Aufnahmebereich (500) an oder in dem Adapter und/oder an oder in einer Konsole (400) befindet, auf der der Adapter angeordnet ist, oder dass der Aufnahmebereich (500) zwischen dem Adapter und der Konsole (400) angeordnet ist.

4. Vorrichtung nach einem der vorhergehenden Ansprüche, **dadurch gekennzeichnet, dass** wenigstens ein Verschiebemechanismus zur Verschiebung der Kanüle (K) vorgesehen ist, der manuell betätigbar ist oder der mit einem Antriebselement in Verbindung steht, das den Verschiebemechanismus betätigt.

5. Vorrichtung nach einem der vorhergehenden Ansprüche, **dadurch gekennzeichnet, dass** das Septum (10) derart angeordnet ist, dass es den Innenraum der Kanüle (K) in der Probennahmeposition gegenüber dem Spülbereich (50) fluiddicht abschließt.

6. Vorrichtung nach einem der vorhergehenden Ansprüche, **dadurch gekennzeichnet, dass** sich in dem Aufnahmebereich (500) das fluidführende System (F) befindet, wobei das fluidführende System (F) seinerseits wenigstens ein Septum (300) aufweist, das in Verschieberichtung der Kanüle (K) mit dem Septum (10) des Adapters fluchtet.

7. Vorrichtung nach Anspruch 6, **dadurch gekennzeichnet, dass** es sich bei dem fluidführenden System (F) um einen Schlauch (200) und vorzugsweise um einen extrakorporalen Blutkreislauf oder um einen Dialysatkreislauf eines Blutbehandlungsgerätes handelt.

8. Vorrichtung nach einem der vorhergehenden Ansprüche, **dadurch gekennzeichnet, dass** die Vorrichtung Mittel zum Aufnehmen einer Fluidprobe aus dem fluidführenden System und/oder wenigstens ein Probennahmegefäß zur Aufnahme einer solchen Fluidprobe und/oder eine Messeinrichtung zur Messung wenigstens eines Parameters des Fluids der Fluidprobe aus dem fluidführenden System aufweist, die unmittelbar oder mittelbar mit dem Innenraum der Kanüle (K) in Fluidverbindung stehen.

9. Vorrichtung nach einem der vorhergehenden Ansprüche, **dadurch gekennzeichnet, dass** der Spülbereich (50) mit Sterilluft, Spüllösung, Kalibrierlösung oder Desinfektionslösung gefüllt ist und/oder dass der Spülbereich (50) mit einem Anschluss in Fluidverbindung steht, der seinerseits mit einer Quelle für Sterilluft, Spüllösung, Kalibrierlösung oder Desinfektionslösung in Verbindung steht oder verbindbar ist.

10. Blutbehandlungsgerät, insbesondere Dialysegerät mit wenigstens einer Vorrichtung nach einem der Ansprüche 1 bis 9.

11. Blutbehandlungsgerät nach Anspruch 10, **dadurch gekennzeichnet, dass** das Gerät wenigstens eine Steuereinheit aufweist, die die Vorrichtung derart ansteuert, dass die Kanüle (K) zum Zwecke der Probennahme in das in den Aufnahmebereich (500) eingelegte fluidführende System (F) eingeführt wird, wobei vorzugsweise vorgesehen ist, dass die Steuereinheit derart ausgebildet ist, dass diese die Probennahme zeitgesteuert oder ereignisgesteuert durchführt.

12. Verfahren zur Probennahme aus einem fluidführenden Schlauchsystem bzw. einen Abschnitt eines Schlauchsystems eines Blutbehandlungsgerätes mittels einer Vorrichtung nach einem der Ansprüche 1 bis 9 oder mittels eines Blutbehandlungsgerätes nach einem der Ansprüche 10 oder 11, **dadurch gekennzeichnet, dass** nach dem Einlegen des fluidführenden Systems (F) in den Aufnahmebereich (500) die Kanüle (K) manuell oder mittels eines Antriebselementes in das fluidführende System (F) verfahren wird und im Anschluss daran eine Probe aus dem fluidführenden System (F) genommen wird.

13. Verfahren nach Anspruch 12, **dadurch gekennzeichnet, dass** das fluidführende System (F) in dem Aufnahmebereich (500) durch eine formschlüssige Verbindung fixiert wird.

14. Verfahren nach einem der Ansprüche 12 oder 13, **dadurch gekennzeichnet, dass** die Probennahme zeit- oder ereignisgesteuert erfolgt.

15. Verfahren nach einem der vorhergehenden Ansprüche, **dadurch gekennzeichnet, dass** nach der Probennahme eine Spülung der Kanüle (K) durchgeführt wird, wobei vorzugsweise vorgesehen ist, das der Spülvorgang automatisch eingeleitet wird.

## Claims

1. Apparatus for sample taking from a fluid-conducting system, **characterized in that** the apparatus has at least one adapter which comprises at least one septum (10) and at least one cannula (K) displaceable relative to the septum (10), wherein the cannula (K) is displaceable such that it penetrates the septum (10) in a sample taking position and does not penetrate the septum (10) in a flushing position, and wherein the adapter has at least one flushing region (50) for a flushing medium which is arranged such that it is in fluid communication with the inner space of the cannula (K) in the flushing position of the cannula (K), and wherein the apparatus has at least one receiving region (500) having locking means for the at least one fluid-conducting system (F) by which the fluid-conducting system (F) is fixable in a defined position relative to the adapter.

2. Apparatus in accordance with claim 1, **characterized in that** the locking means fix the fluid-conducting system (F) relative to the adapter by form-fit.

3. Apparatus in accordance with claim 1 or claim 2, **characterized in that** the receiving region (500) is located at or in the adapter and/or at or in a console (400) on which the adapter is arranged; or **in that** the receiving region (500) is arranged between the adapter and the console (400).

4. Apparatus in accordance with one of the preceding claims, **characterized in that** at least one displacement mechanism for displacing the cannula (K) is provided, which is manually actuable or which is connected to a drive element which actuates the displacement mechanism.

5. Apparatus in accordance with one of the preceding claims, **characterized in that** the septum (10) is arranged such that it terminates the inner space of the cannula (K) in a fluid-tight manner with respect to the flushing region (50) in the sample taking position.

6. Apparatus in accordance with one of the preceding claims, **characterized in that** the fluid-conducting system (F) is located in the receiving region (500), wherein the fluid-conducting system (F) in turn has at least one septum (300) which is aligned with the septum (10) of the adapter in the displacement direction of the cannula (K).

7. Apparatus in accordance with claim 6, **characterized in that** the fluid-conducting system (F) is a hose (200) and preferably an extracorporeal blood circuit or a dialyzate circuit of a blood treatment device.

8. Apparatus in accordance with one of the preceding claims, **characterized in that** the apparatus has means for receiving a fluid sample from the fluid-conducting system and/or has at least one sample taking vessel for receiving such a fluid sample and/or has a measuring device for measuring at least one parameter of the fluid of the fluid sample from the fluid-conducting system which are directly or indirectly in fluid communication with the inner space of the cannula (K).

9. Apparatus in accordance with one of the preceding claims, **characterized in that** the flushing region (50) is filled with sterile air, flushing solution, calibration solution or disinfecting solution, and/or **in that** the flushing region (50) is in fluid communication with a connector which is in turn connected or connectable to a source for sterile air, flushing solution, calibration solution or disinfecting solution.

10. Blood treatment device, in particular a dialysis device, having at least one apparatus in accordance with one of the claims 1 to 9.

11. Blood treatment device in accordance with claim 10, **characterized in that** the device has at least one control unit which controls the apparatus such that the cannula (K) is introduced into the fluid-conducting system (F) inserted into the receiving region (500) for the purpose of the sample taking, wherein preferably provision is made that the control unit is configured such that is carries out the sample taking in a time-controlled or event-controlled manner.

12. Method for taking a sample from a fluid-conducting hose system or a section of a hose system of a blood treatment device by means of an apparatus in accordance with one of the claims 1 to 9 or by means of a blood treatment device in accordance with one of the claims 10 or 11, **characterized in that** the cannula (K) is moved manually or by means of a drive element into the fluid-conducting system (F) after the placement of the fluid-conducting system (F) into the receiving region (500) and subsequent to this a sample is taken from the fluid-conducting system (F).

13. Method in accordance with claim 12, **characterized in that** the fluid-conducting system (F) is fixed by a form-fit connection in the receiving region (500).

14. Method in accordance with one of the claims 12 or 13, **characterized in that** the sample taking takes place in a time- or event controlled manner.

15. Method in accordance with one of the preceding claims, **characterized in that** a flushing of the cannula (K) is carried out after the sample taking, wherein preferably provision is made that the flushing procedure is automatically initiated.

## Revendications

1. Dispositif de prélèvement d'échantillons d'un système d'acheminement de fluide, **caractérisé en ce que** le dispositif présente au moins un adaptateur, qui comprend au moins un septum (10) et au moins une canule (K) pouvant être coulissée par rapport au septum (10), dans lequel la canule (K) peut être coulissée de telle manière que celle-ci traverse, dans une position de prélèvement d'échantillons, le septum (10) et ne traverse pas, dans une position de rinçage, le septum (10) et dans lequel l'adaptateur présente au moins une zone de rinçage (50) pour un milieu de rinçage, qui est disposée de telle sorte que celle-ci se trouve en communication fluidique avec l'espace intérieur de la canule (K) dans la position de rinçage de la canule (K), et dans lequel le dispositif présente au moins une zone de réception (500) avec des moyens d'arrêt pour l'au moins un système d'acheminement de fluide (F), par lesquels le système d'acheminement de fluide (F) peut être bloqué par rapport à l'adaptateur dans une position définie.

2. Dispositif selon la revendication 1, **caractérisé en ce que** les moyens d'arrêt bloquent par complémentarité de forme le système d'acheminement de fluide (F) par rapport à l'adaptateur.

3. Dispositif selon la revendication 1 ou 2, **caractérisé en ce que** la zone de réception (500) se trouve au niveau du ou dans l'adaptateur et/ou se trouve au niveau d'une ou dans une console (400), sur laquelle est disposé l'adaptateur, ou que la zone de réception (500) est disposée entre l'adaptateur et la console (400).

4. Dispositif selon l'une quelconque des revendications précédentes, **caractérisé en ce qu'**au moins un mécanisme de coulissement est prévu pour faire coulisser la canule (K), lequel peut être actionné manuellement ou est relié à un élément d'entraînement, qui actionne le mécanisme de coulissement.

5. Dispositif selon l'une quelconque des revendications précédentes, **caractérisé en ce que** le septum (10) est disposé de telle manière qu'il ferme de manière étanche fluidiquement l'espace intérieur de la canule (K) dans la position de prélèvement d'échantillons par rapport à la zone de rinçage (50).

6. Dispositif selon l'une quelconque des revendications précédentes, **caractérisé en ce que** le système d'acheminement de fluide (F) se trouve dans la zone de réception (500), dans lequel le système d'acheminement de fluide (F) présente quant à lui au moins un septum (300), qui est en affleurement avec le septum (10) de l'adaptateur dans la direction de coulissement de la canule (K).

7. Dispositif selon la revendication 6, **caractérisé en ce que** le système d'acheminement de fluide (F) est un tuyau flexible (200) et de préférence un circuit de sang extracorporel ou un circuit de dialysat d'un appareil de traitement du sang.

8. Dispositif selon l'une quelconque des revendications précédentes, **caractérisé en ce que** le dispositif présente des moyens servant à recevoir un échantillon de fluide provenant du système d'acheminement de fluide et/ou au moins un récipient de prélèvement d'échantillons servant à recevoir un échantillon de fluide de ce type et/ou un système de mesure servant à mesurer au moins un paramètre du fluide de l'échantillon de fluide provenant du système d'acheminement de fluide, qui sont en communication fluidique directement ou indirectement avec l'espace intérieur de la canule (K).

9. Dispositif selon l'une quelconque des revendications précédentes, **caractérisé en ce que** la zone de rinçage (50) est remplie d'air stérile, de solution de rinçage, de solution d'étalonnage ou de solution de désinfection, et/ou que la zone de rinçage (50) est en communication fluidique avec un raccord, qui quant à lui est relié ou peut être relié à une source d'air stérile, de solution de rinçage, de solution d'étalonnage ou de solution de désinfection.

10. Appareil de traitement du sang, en particulier appareil de dialyse, avec au moins un dispositif selon l'une quelconque des revendications 1 à 9.

11. Appareil de traitement du sang selon la revendication 10, **caractérisé en ce que** l'appareil présente au moins une unité de commande, qui pilote le dispositif de telle manière que la canule (K) est introduite dans le système d'acheminement de fluide (F) placé dans la zone de réception (500) aux fins du prélèvement d'échantillons, dans lequel il est prévu de préférence que l'unité de commande est réalisée de telle manière que celle-ci exécute le prélèvement d'échantillons par une commande dans le temps ou par une commande par un événement.

12. Procédé de prélèvement d'échantillons provenant un système de tuyau flexible d'acheminement de fluide ou d'une section d'un système de tuyau flexible d'un appareil de traitement du sang au moyen d'un dispositif selon l'une quelconque des revendications 1 à 9 ou au moyen d'un appareil de traitement du sang selon l'une quelconque des revendications 10 ou 11, **caractérisé en ce qu'**après le placement du système d'acheminement de fluide (F) dans la zone de réception (500), la canule (K) est déplacée manuellement ou au moyen d'un élément d'entraînement dans le système d'acheminement de fluide (F) puis un échantillon est prélevé directement après du système d'acheminement de fluide (F).

13. Procédé selon la revendication 12, **caractérisé en ce que** le système d'acheminement de fluide (F) est bloqué par une liaison à complémentarité de forme dans la zone de réception (500).

14. Procédé selon l'une quelconque des revendications 12 ou 13, **caractérisé en ce que** le prélèvement d'échantillons est effectué par une commande dans le temps ou par une commande par un événement.

15. Procédé selon l'une quelconque des revendications précédentes, **caractérisé en ce qu'**après le prélèvement d'échantillons, un rinçage de la canule (K) est mis en œuvre, dans lequel il est prévu de préférence que l'opération de rinçage est initiée automatiquement.
